# EUROPEAN PATENT APPLICATION

(11) **EP 4 245 206 A1**
(43) Date of publication of application: **20.09.2023**
(21) Application number: 23157976.4
(22) Date of filing: 22.02.2023
(51) Int. Cl.: A61B 1/00, A61B 1/015, A61B 1/018, A61B 1/12

(54) **HAND-HELD SURGICAL INSTRUMENT AND MAIN BODY FOR HAND-HELD SURGICAL INSTRUMENT**

(30) Priority: 15.03.2022 US 202263319827 P
(71) Applicant: Olympus Winter & Ibe GmbH, 22045 Hamburg (DE)
(72) Inventor: Schmuck, Irina, 25746 Heide (DE); Schröder, Bastian, 22589 Hamburg (DE); Schulz, Kevin Alexander, 22889 Wilstedt (DE); Rühs, Andreas, 22926 Ahrensburg (DE)
(74) Representative: Hoener, Matthias

(57) **Abstract**

The invention provides a main body (11) for a hand-held surgical instrument (10) and a hand-held surgical instrument (10) in which tools or through articles can be passed through a channel (34) in a simple manner and with which a sufficiently large flushing flow can be passed through the channel (34) at the same time. This is achieved by having two rinsing holes (23), which extend through a wall (29) of a main body (11) of a hand-held surgical instrument (10), directed towards an upper region (24) of a cross-section of a channel (34) extending axially through the main body (11). In this regard, the holes (23) are arranged transversely to the channel (34).

## Description

The invention relates to a main body for a hand-held surgical instrument according to claim 1. Furthermore, the invention relates to a hand-held surgical instrument according to claim 11.

Hand-held surgical instruments, such as endoscopes, cystoscopes and resectoscopes, are known to be used for urological applications. Some treatments or operations require the area to be treated inside the patient's body to be filled with a fluid. For example, before the human bladder is treated, it is filled with water. In addition, it is known that when optical instruments are used, the distal area of the instrument is surrounded by a liquid during treatment in order to create the best possible viewing conditions for the surgeon. In these applications, it is particularly important that the fluid flow through the instrument, especially through a shaft of the instrument, into the body takes place at a constant as well as sufficiently high flow velocity. For this purpose, it is provided in the above-mentioned instruments that two rinsing connections are arranged on a main body of the instruments. Via these rinsing connections, a liquid can be directed through rinsing bores in the main body into a channel or into an inner shaft of the instrument. From there, the rinsing liquid can be guided through a shaft connectable to the main body to the place of treatment or into an inner space of the human body.

The rinsing bores extend through a wall of the main body and meet the channel laterally. The channel typically has a circular cross-section and extends axially through the main body. For example, an optic can be pushed through the channel from the proximal end to the distal end of the shaft. It is known that a working channel can also extend through the main body, in particular parallel to the channel. This working channel is used for receiving or passing through tools and/or through articles, such as guide wires or electrical conductors. Said tools and/or through articles are guided from the proximal side of the instrument through the working channel of the main body and optionally through a shaft to the distal end of the instrument, where they are used for treatment.

In known applications, the necessary force to pass the liquid through the instrument is generated by hydrostatic pressure. For this purpose, a bag containing the liquid is suspended at a sufficient height above the patient and connected to the rinsing bores/ports via a hose. Further regulation of the flow rate can be generated by valves located at the ports. The rinsing bores can also be used to clean or reprocess the instrument after treatment using an irrigation fluid. Other components can also be connected to the main body of the instrument, such as a bridge, a handle unit, an optics plate or the like.

Passing the tools or through articles, such as guide wires, through the working channel of the main body has proven to be problematic with these instruments. In particular, the through articles can get caught or snagged in the rinsing bores when passing through the working channel. This makes it more difficult to guide the tools and through articles through to the distal end of the instrument.

Based thereon, the object of the present invention is to provide a main body for a hand-held surgical instrument and a hand-held surgical instrument in which tools or through articles can be passed through a channel in a simple manner and with which a sufficiently large irrigation flow can be passed through the channel at the same time.

A main body for solving this task has the features of claim 1. Accordingly, it is provided that two rinsing bores extending through a wall of a main body of a hand-held surgical instrument are directed towards an upper region of a cross-section of a channel extending axially through the main body. Thereby, the bores are arranged transversely, preferably at a 90° angle, to the channel. An optic or an inner shaft is passed through the upper cross-sectional region of the channel during use of the instrument. The lower cross-sectional region of the channel is separated from the upper cross-sectional region by the optic or other inner shaft that is pushed through the channel. This lower cross-sectional region is also referred to as the working channel. Similarly, the upper cross-sectional region may be referred to as the optics channel.

Tools or through articles are fed through the working channel. By positioning the rinsing bores or the outlets of the rinsing bores in an upper region of the cross-section of the channel, the fluid flowing through the rinsing bores hits the optics or the inner shaft located inside the channel. The optics or inner shaft covers the rinsing bores towards the lower region of the cross-section of the channel or towards the working channel. When the tools or through articles are fed through the working channel in this state, they can no longer get caught in the rinsing bores. This provides a simple as well as reliable way to insert the tool or the through articles into the main body without interference.

Preferably, the invention further provides that the two rinsing bores enclose an angle of not equal to 180° with respect to each other. Preferably, it is also conceivable that the two rinsing bores enclose an angle of from 175° to 90°, in particular from 170° to 135° or from 160° to 145°, with respect to each other. A particularly advantageous embodiment may provide that the two bores enclose an angle of 150° with respect to each other. This obtuse angle between the two bores causes the flushing fluid to strike an upper portion of the tube-like optic or inner shaft at an angle. On the one hand, this can more efficiently prevent the through articles from getting caught in the bores. On the other hand, this oblique impingement of the rinsing liquid on the inner shaft improves the inflow behavior of the liquid into the interior of the main body. Ultimately, the rinsing fluid should be guided through the channel to the distal end of the instrument at a large flow rate. This can be achieved in particular by inclining the rinsing bores relative to a horizontal plane.

Preferably, the invention further provides that the two rinsing bores are brought together above the working channel and form a clearance there. This clearance represents an enlargement of the channel or the interior of the main body. This clearance or enlargement is formed directly above the optics or inner shaft, so that the rinsing liquid flowing through the rinsing bores into the interior of the main body finds sufficient space to flow there around the optics or inner shaft and through the channel, in particular through the working channel, to the distal end of the instrument. Ultimately, this increased space allows the necessary flow rate to be achieved to both flood the treatment space inside the patient with the fluid and to flush the area around the distal end of the instrument clear.

In particular, it is intended that the clearance extends in the distal and/or proximal direction within the main body at least in sections along the channel. Depending on the embodiment of the main body, the clearance may be limited to a very small area or may be formed over a large part of the length of the channel. Ultimately, the main body also serves to guide aforementioned tools and shanks. Therefore, the channel or the working channel is designed precisely in such a way that it serves as a guide for the aforementioned tools and shafts and, at the same time, has a geometry or cross-section that is particularly suitable for the inflow of the flushing liquid.

A particularly advantageous embodiment of the invention can provide that the channel has an elongated, in particular oval, cross-section, whereby a rod-shaped optic or an inner shaft can be guided through an upper region of the cross-section and through a lower region of the cross-section, the working channel, through articles. It is also conceivable that the elongated cross-section of the channel has the contours of a figure eight. In this embodiment, the optics or the inner shaft is guided through the upper area and the through articles through the lower area. Due to the waisted cross-section, the working channel or the lower area of the cross-section is ideally shielded from the rinsing bores, so that the possibility of the through articles getting caught in the bores can be ruled out.

It is also conceivable that the cross-section of the working channel is enlarged in a funnel-like manner in the proximal direction of the main body for the introduction of through articles. This funnel-like enlargement serves to improve or even facilitate the introduction of the through articles into the lower cross-sectional area of the working channel.

Another embodiment may provide that an outer tube, for example a shaft or the like, is couplable to a distal end of the main body and further components of the instrument are releasably couplable to a proximal end. These components may be, for example, a bridge. This bridge can also have at least one channel, in particular a working channel, which is used to pass tools, optics and/or through articles. The bridge may have a distal end face through which the channel, clearance and funnel-like enlargement of the working channel of the main body are bounded in the proximal direction.

A hand-held surgical instrument, in particular an endoscope, a cystoscope, a resectoscope or the like, for solving said task has the features of claim 11. Accordingly, it is provided that the hand-held surgical instrument comprises a main body according to at least one of claims 1 to 10.

A preferred embodiment of the invention is explained in more detail below with reference to the drawing. In this show:
- Fig. 1: A schematic representation of a hand-held surgical instrument,
- Fig. 2: A sectional view through a main body of a hand-held surgical instrument,
- Fig. 3: A schematic representation according to Fig. 2,
- Fig. 4: An enlarged view of the sectional view according to Fig. 2,
- Fig. 5: A sectional view through the main body of the hand-held surgery instrument, and
- Fig. 6: Another illustration of the section according to Fig. 5.

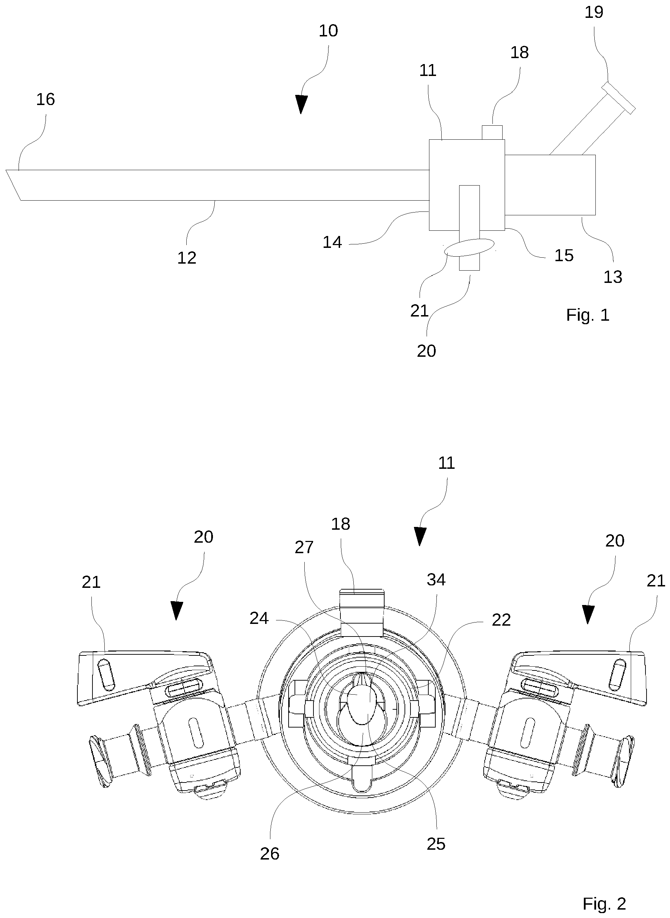

Fig. 1 is a highly schematized illustration of a hand-held surgical instrument 10. It should be explicitly noted that this illustration is intended merely to illustrate the subject matter of the invention and therefore any further details and realistic dimensions of a hand-held surgical instrument have been omitted. For a description of a hand-held surgical instrument, reference is made to the known prior art.

The instrument 10 outlined herein may be an endoscope, a cystoscope, a resectoscope or the like. The essential components of the instrument 10 are formed by a main body 11, a shaft 12 and a bridge 13. While the shaft 12 can be coupled to a distal side 14 of the main body 11, the bridge 13 can be coupled to a proximal side 15 of the main body 11. In the embodiment example shown in Fig. 1, for example, an actuating means 18 is arranged on the main body 11 to cancel the coupling with the bridge 13. In addition, it is conceivable that in particular the shaft 12 and the bridge 13 can be releasably connected to the main body 11 via other types of connection.

During treatment, the instrument 10 is inserted with the distal end 16 of the shaft 12 leading into the patient's body part to be treated. A tube-like channel 34 extends axially within the shaft 12, the main body 11 and the bridge 13, through which an optic 28, tools or other passageway items, such as guide wires or electrical conductors, can be guided to the distal end 16 of the shaft 12. With the aid of these tools, the surgeon can target the treatment. The example embodiment of the instrument 10 shown in Fig. 1 includes an ocular 19 on the bridge 13, which is connected to an optical fiber within the channel 34 of the instrument 10. Via this ocular 19, the operator can directly view the treatment area. Alternatively, it is also conceivable that a digital camera is arranged on the bridge 13 to display the treatment on a screen. Handle units, mechanical feedthroughs, electrical feedthroughs, sockets for electrical contacting of a tool or the like can also be arranged on the bridge 13.

For many treatments, it is necessary for the area in front of the distal end 16 of the instrument 10 to be rinsed by a flushing liquid. This rinsing improves visibility for the surgeon and, if necessary, fills a cavity in the body to be treated with fluid. The irrigation fluid can be introduced into the working channel 17 via an rinsing connection 20 on the main body 11. In the embodiment example of the instrument 10 shown in Fig. 1, a rinsing connection 20 is shown in a highly schematized manner on the side of the main body 11. It is quite common for another rinsing connection 20 to be arranged on an opposite side of the main body 11. The flushing flow can be regulated by means of a cock 21 at the rinsing connection 20. In order to supply a sufficient amount of flushing liquid, the rinsing connection 20 can be connected to a liquid reservoir. The other end of the rinsing connection 20 is led through a wall 22 into a rinsing bore 23, which opens into the channel 34. Flushing fluid flowing through the rinsing ports 20 enters the channel 34 through the rinsing bore 23 and is directed to the distal end 16 of the shaft 12 by appropriate pressurization. In addition to being used during treatment, the rinsing fluid may also be used for subsequent cleaning or reprocessing purposes.

Fig. 2 shows a section through the main body 11 perpendicular to the longitudinal axis of the instrument 10. Running centrally through the middle of the main body 11 is the channel 34. The cross-section of the working channel 17 is oval. While the upper region 24 of the channel 34 is used to receive an inner shaft or optic 28, through articles such as guide wires or other electrical conductors can be passed through a lower region 25. This lower region 25 is also referred to as the working channel 17. For ease of insertion of the aforementioned instruments, the channel 34 has a funnel-like receptacle 26 in the proximal direction. This receptacle 26 is shaped to guide the aforementioned tools directly into the working channel 17.

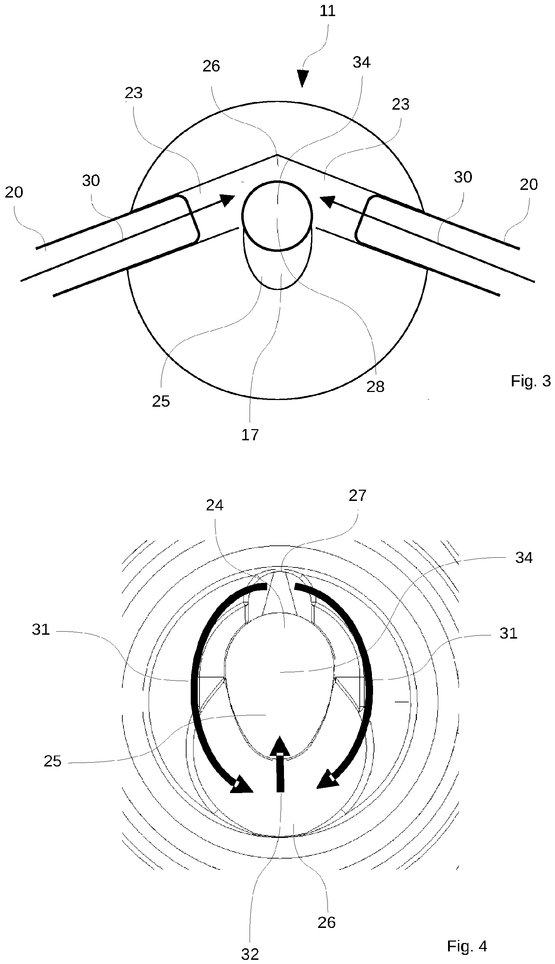

Above the channel 34, a clearance 27 can be seen in Fig. 2. This clearance 27 is a free volume which is located above the channel 34 within the main body 11 and serves for rinsing the optics or the inflow of the rinsing liquid into the channel 34. To the side of the main body 11 are shown the two rinsing connections 20. From this illustration, it is very clear that the two rinsing connections 20 enclose an obtuse angle and thus the inflowing rinsing liquid enters the channel 34 at a corresponding angle. Thus, the flushing fluid does not strike the optics 28 or the inner shaft, which is not shown, in the upper portion 24 of the channel 34 perpendicularly, but at an angle different from 90°. For illustrative purposes, this relative orientation of the rinsing connections 20 is shown in highly schematized form in Fig. 3. Also shown in this figure is a cross-section through the centrally located channel 34 of the main body 11. In the upper region 24 of the channel 34, a rod-like optic 28 is carried out. Therefore, this region 24 may also be referred to as an optics channel. This optic 28 may be a rod lens system or an optical fiber. From the sides of the main body 11, two rinsing bores 23 are led through the wall 29 of the main body 11. In these rinsing bores 23, connecting pieces of the rinsing connections 20 are schematically shown. The rinsing bores 23 are led into the main body 11 so far that they meet above the channel 34 and form the clearance 27 there. If the rinsing liquid now enters the rinsing bores 23 through the rinsing connections 20 according to the direction of arrow 30, the optics 28 are rinsed around. Due to the clearance 27 extending in the proximal direction, the rinsing liquid can also flow around the optics 28 into the lower area 25 or into the working channel 17. From here, the rinsing fluid flows in a distal direction to the distal end 16 of the shaft 12.

An essential feature of the invention is that the rinsing bores 23 or the flushing liquid meets the upper region 24 or the optics 28 at an upper region and flows together in the clearance 27. It also proves to be particularly advantageous that the optic 28 or an inner shaft, which is arranged in the upper region 24 of the channel 34, conceals a direct connection between the working channel 17 and the rinsing bores 23. As a result, the through articles cannot get caught in the rinsing bores 23 when inserted into the working channel 17. Such snagging can cause damage to the through articles as well as delays in treatment preparation. The working channel 17 is thus covered by the optics 28 or an inner shaft which is guided through the upper region 24. It should again be expressly noted that the illustration according to Fig. 3 serves only to describe the invention and may differ from the actual design of the main body 11.

Fig. 4 shows an enlarged section of Fig. 2. In this Fig. 4, the arrows 31 show the direction of flow of the rinsing liquid from the clearance 27 in the direction of the funnel-like receptacle 26. Thereby, rinsing fluid in clearance 27 flows around channel 34 into the area of receptacle 26. From there, the rinsing fluid flows in the direction of arrow 32 through channel 34 or working channel 17 in the distal direction of instrument 10. The rinsing bores 23 are not visible in this sectional view.

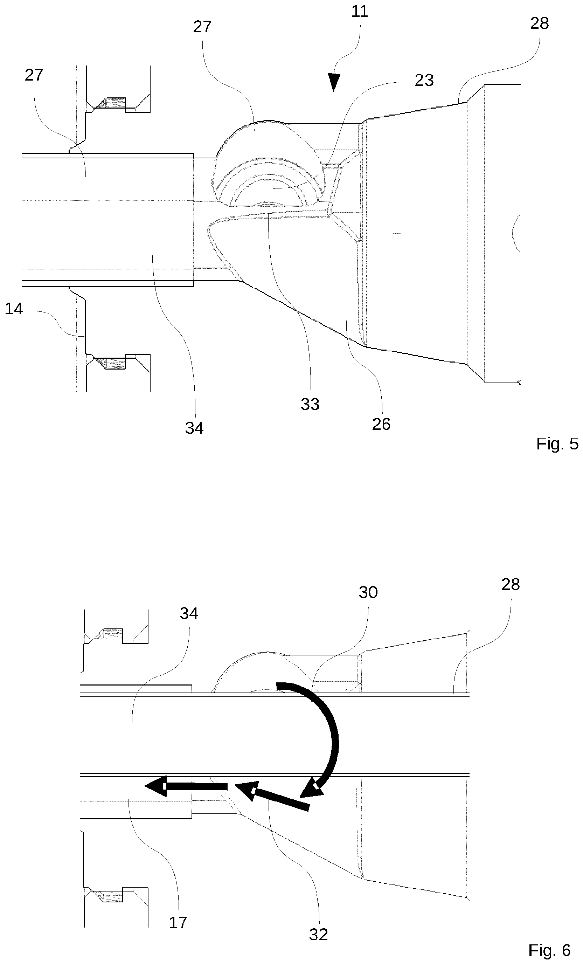

Figs. 5 and 6 each show sectional views transverse to the sections of Figs. 2 and 4 through the main body 11. While in Fig. 6 an optic 28 is guided through the channel 34, this optic 28 is missing in Fig. 5. In Fig. 5, the view is unobstructed to a rinsing bores 23 and the adjacent clearance 27. In the lower area of the main body 11, the funnel-like receptacle 26 can be seen, which is separated from the rinsing bores 23 by a bar 33. This bar 33 holds the optic 28 or an inner shaft in position in the upper region 24 of the channel 34, and prevents through articles not shown from getting caught in the rinsing bores 23. In the proximal direction, the bridge 13 joins the main body 11. On the opposite distal side 14, the shaft 12 can be coupled.

Fig. 6 shows how the rinsing liquid flows around the optics 28 in the direction of arrow 30 in the clearance 27 and flows out of the receptacle 26 in the direction of arrow 32 in the distal direction. The rinsing liquid can also flow between the optics 28 and the wall of the working channel 17.

It should be expressly noted that the embodiment example shown in the figures represents only one possible embodiment of the main body according to the invention. Furthermore, it is conceivable that the claimed main body can also be formed in alternative embodiments.

### List of Reference Numerals:

- 10: Hand-held surgical instrument
- 11: Main body
- 12: Shaft
- 13: Bridge
- 14: Distal side
- 15: Proximal side
- 16: Distal end
- 17: Working channel
- 18: Actuating means
- 19: Ocular
- 20: Rinsing connection
- 21: Cock
- 22: Wall
- 23: Rinsing bores
- 24: Upper region
- 25: Lower region
- 26: receptacle
- 27: Clearance
- 28: Optics
- 29: Wall
- 30: Arrow direction
- 31: Arrow
- 32: Arrow direction
- 33: Bar
- 34: Channel

## Claims

1. Main body (11) for a hand-held surgical instrument (10), in particular for an endoscope, a cystoscope, a resectoscope or the like, with a channel (34) extending axially through the main body (11) for the passage of tools, an optical system (28) and/or through articles, in particular guide wires or electrical conductors, and with two rinsing bores (23) for connecting a respective rinsing connection (20), wherein the rinsing bores (23) extend from two opposite sides of the main body (11) through a wall (29) of the main body (11) towards the channel (34), **characterised in that** the rinsing bores (23) are aligned with an upper region (24) of a cross-section of the channel (34).

2. Main body (11) for a hand-held surgical instrument (10) according to claim 1, **characterized in that** the two rinsing bores (23) enclose an angle of unequal 180° to each other.

3. Main body (11) for a hand-held surgical instrument (10) according to claim 1, **characterized in that** the two rinsing bores (23) enclose an angle of 175° to 90°, in particular of 170° to 135° or of 160° to 145°, preferably of 150°, with respect to each other.

4. Main body (11) for a hand-held surgical instrument (10) according to one of the previous claims, **characterized in that** the two rinsing bores (23) are brought together above the channel (34) and form a clearance (27) there.

5. Main body (11) for a hand-held surgical instrument (10) according to claim 4, **characterized in that** the clearance (27) extends in distal and/or proximal direction within the main body (11), at least in sections along the channel (34).

6. Main body (11) for a hand-held surgical instrument (10) according to one of the previous claims, **characterized in that** the channel (34) has an elongated, in particular oval, cross-section, wherein through an upper region (24) of the cross-section, in particular an optics channel, a rod-shaped optic (28) or an inner shaft and through a lower region (25) of the cross-section, in particular a working channel (17), through articles can be guided.

7. Main body (11) for a hand-held surgical instrument (10) according to one of the previous claims, **characterized in that** the rinsing bores (23) open into the upper region (24) of the cross-section and in particular a free access from the lower region (25) of the cross-section to the rinsing bores (23) is covered by an optic (28) or an inner shaft which can be guided through the upper region of the cross-section of the cross-section

8. Main body (11) for a hand-held surgical instrument (10) according to one of the previous claims, **characterized in that** a lower region (25) of the channel (34), in particular the working channel (17), in the proximal direction of the main body (11) for the introduction of through articles increases in its cross-section funnel-like to a receptacle (26).

9. Main body (11) for a hand-held surgical instrument (10) according to any one of the preceding claims, **characterized by** a distal end to which an outer tube, a shaft (12) or the like can be coupled and by a proximal end to which further components of the instrument can be coupled.

10. Main body (11) for a hand-held surgical instrument (10) according to one of the previous claims, **characterized in that** a bridge (13) can be coupled to the proximal end of the main body (11), wherein the bridge (13) has at least one channel, in particular a working channel, for the passage of tools, an optical system and/or through articles, and wherein the bridge (13) has a distal end face by which the channel (34), the clearance (27) and the funnel-like receptacle (26) of the working channel (17) of the main body (11) are delimited in the proximal direction.

11. Hand-held surgical instrument (10), in particular an endoscope, a cystoscope, a resectoscope or the like, comprising a main body (11) according to any one of claims 1 to 10.
